# EUROPÄISCHE PATENTANMELDUNG

(11) **EP 1 086 655 A1**
(43) Veröffentlichungstag der Anmeldung: **28.03.2001**
(21) Anmeldenummer: 00115433.5
(22) Anmeldetag: 18.07.2000
(51) Int. Cl.: A61B 17/17, A61B 17/72

(54) **Repositionsinstrumentarium zur Fixation von Knochenfrakturen**

(30) Priorität: 23.09.1999 DE 19945612
(71) Anmelder: AESCULAP AG, 78532 Tuttlingen (DE)
(72) Erfinder: Stedtfeld, Hans-Werner, 90475 Nürnberg (DE); Böttiger, Roland, 78604 Rietheim-Weilheim (DE); Saueressig,Thomas, 78532 Tuttlingen (DE)
(74) Vertreter: Böhme, Ulrich, Dr. Dipl.-Phys.

(57) **Zusammenfassung**

Um bei einem Repositionsinstrumentarium für Fragmentfrakturen von Röhrenknochen mit einem in den Röhrennochen einführbaren, schaftförmigen, querverlaufende Fixationsbohrungen aufweisenden Nagel und mit einem mit diesem über Fixierelemente verbindbaren Zielgerät eine Reposition auch durchführen zu können, ohne in Längsrichtung des Fixationsnagels Knochenmaterial entfernen zu müssen, wird vorgeschlagen, daß mehrere Fixationsbohrungen mit einem Innengewinde versehen sind, in welches stiftförmige Fixierelemente reproduzierbar weit einschraubbar sind, daß das Zielgerät zur im wesentichen spielfreien Aufnahme derartiger Fixierelemente mehrere Durchgangsbohrungen aufweist, daß die Fixationsbohrungen und die Durchgangsbohrungen so angeordnet sind, daß bei einer bestimmten Position des Zielgerätes relativ zum Fixationsnagel jeweils eine Fixationsbohrung und eine Durchgangsbohrung miteinander ausgerichtet sind, daß die Fixierelemente in den Durchgangsbohrungen in axialer Richtung derart festlegbar sind, daß das Zielgerät bei in die Fixationsbohrungen eingeschraubten Fixierelementen in dieser bestimmten Position relativ zum Fixationsnagel fixierbar ist, und daß die stiftförmigen Fixierelemente nach Lösung dieser Fixierung und Lösung der Gewindeverbindung mit den Fixationsbohrungen aus den Durchgangsbohrungen des Zielgeräts herausziehbar sind.

## Beschreibung

Die Erfindung betrifft ein Repositionsinstrumentarium für Fragmentfrakturen von Röhrenknochen mit einem in den Röhrenknochen einführbaren, schaftförmigen, querverlaufende Fixationsbohrungen aufweisenden Fixationsnagel und mit einem mit diesem über Fixierelemente verbindbaren Zielgerät.

Bei der Versorgung komplexer Fragmentfrakturen im Bereich des Kopfes eines Röhrenknochens, insbesondere des Humerusknochens, ist es üblich, in den Röhrenknochen einen schaftförmigen Fixationsnagel einzuführen, der Querbohrungen aufnimmt, durch die Knochenschrauben hindurchgeführt werden, mit denen die Knochenfragmente am Fixationsnagel befestigt werden.

Die dazu notwendigen Bohrungen in den Knochenfragmenten werden vorzugsweise mittels eines Zielgerätes gesetzt, welches mit dem Fixationsnagel verbunden ist und dem Operateur die Richtung vorgibt, die der Richtung der Fixationsbohrungen entspricht.

Bei Kopffrakturen ergeben sich jedoch dann Schwierigkeiten, wenn der Kopf aus den Knochenfragmenten vollständig aufgebaut werden soll, da ohne eine Öffnung im Kopf diese Zielgeräte bei bekannten Fixationsnägeln nicht mit dem Fixationsnagel verbunden werden können, die Verbindung erfolgt üblicherweise in Richtung der Längsachse des Fixationsnagels über dort angreifende Fixierelemente.

Es ist Aufgabe der Erfindung, ein Repositionsinstrumentarium insgesamt so auszubilden, daß auch ohne eine Öffnung in dem zu rekonstruierenden Kopfteil ein Zielgerät am Fixationsnagel festgelegt werden kann, mit welchem es möglich ist, in die einzelnen Knochenfragmente mit den Fixationsbohrungen im Fixationsnagel ausgerichtete Bohrungen für Knochenschrauben einzubringen.

Diese Aufgabe wird bei einem Repositionsinstrumentarium der eingangs beschriebenen Art erfindungsgemäß dadurch gelöst, daß in mehrere Fixationsbohrungen stiftförmige Fixierelemente reproduzierbar weit einsetzbar und in dieser Stellung festlegbar sind, daß das Zielgerät zur im wesentlichen spielfreien Aufnahme derartiger Fixierelemente mehrere Durchgangsbohrungen aufweist, daß die Fixationsbohrungen und die Durchgangsbohrungen so angeordnet sind, daß bei einer bestimmten Position des Zielgerätes relativ zum Fixationsnagel gleichzeitig jeweils eine Fixationsbohrung und eine Durchgangsbohrung miteinander ausgerichtet sind, daß die Fixierelemente in den Durchgangsbohrungen in axialer Richtung derart festlegbar sind, daß das Zielgerät bei in die Fixationsbohrungen eingesetzten und in diesen festgelegten Fixierelementen in dieser bestimmten Position relativ zum Fixationsnagel fixierbar ist, und daß die stiftförmigen Fixierelemente nach Lösung dieser Fixierung und Lösung der Festlegung in den Fixationsbohrungen aus den Durchgangsbohrungen des Zielgeräts herausziehbar sind.

Eine solche Ausgestaltung ermöglicht es, durch die in die Fixationsbohrungen eingesetzten Fixierelemente das Zielgerät seitlich des Röhrenknochens anzuordnen, die Fixierelemente verlaufen dabei im wesentlichen radial relativ zum Fixationsnagel. Dadurch, daß mehrere derartige Fixationsbohrungen und damit ausrichtbare Durchgangsbohrungen im Zielgerät vorgesehen sind, können diese Paare von Fixationsbohrungen und Durchgangsbohrungen nacheinander mit Fixierelementen versehen werden, dabei genügen im Prinzip zwei Fixierelemente, um eine eindeutige und sichere Fixierung des Zielgerätes relativ zum Fixationsnagel zu ermöglichen, die übrigen Paare von Durchgangsbohrungen und Fixationsbohrungen stehen dann zum Einbringen von Bohrungen in den zu repositionierenden Knochenfragmenten zur Verfügung, und zwar unter exakter Ausrichtung des Bohrers, da die Durchgangsbohrungen als Zielbohrungen verwendet werden.

Dabei ist es möglich, nacheinander unterschiedliche Paare von Fixationsbohrungen und Durchgangsbohrungen mit Fixierelementen zu belegen, so daß durch diesen Wechsel nacheinander unterschiedliche Paare von Durchgangsbohrungen und Fixationsbohrungen frei werden zum Setzen von Knochenbohrungen. Dies ist möglich, weil die Fixierelemente nach dem Lösen der Festlegung in den Fixationsbohrungen und nach dem Lösen der axialen Fixierung im Zielgerät nach hinten aus dem Zielgerät herausziehbar sind.

Das Zielgerät wird also über Fixierelemente relativ zum Fixationsnagel fixiert, die ihre Position im Verlauf der Operation ändern und die es dem Operateur damit ermöglichen, in unterschiedlichen Operationsbereichen tätig zu werden. Trotzdem bleiben das Zielgerät und der Fixationsnagel während der gesamten Operation in der bestimmten Position relativ zueinander, die gewährleistet, daß die Durchgangsbohrungen und die entsprechende Fixationsbohrung paarweise zueinander ausgerichtet sind.

Besonders vorteilhaft ist es, wenn im proximalen Endbereich des Fixationsnagels in verschiedenem Abstand vom proximalen Ende des Fixationsnagels in unterschiedliche Richtungen weisende Fixationsbohrungen vorgesehen sind, denen jeweils eine mit ihr ausrichtbare Durchgangsbohrung des Zielgerätes zugeordnet sind. Diese Fixationsbohrungen am proximalen Endbereich des Fixationsnagels ermöglichen es, die Fixierelemente im proximalen Endbereich des Röhrenknochens anzubringen, also zwischen den seitlich abklappbaren Frakturfragmenten, dadurch ist eine Verbindung zwischen Zielgerät und Fixationsnagel möglich, ohne Knochenteile entfernen zu müssen, also beispielsweise ohne ein Aufbohren des Knochenkopfes, die Fixiernägel können zwischen den Knochenfragmenten hindurchgesteckt werden.

Insbesondere kann vorgesehen sein, daß zwei der Fixationsbohrungen in einer Ebene liegen und die anderen beiden schräg zu dieser Ebene verlaufen.

Dabei ist es günstig, wenn der Winkel zwischen den schräg zu der Ebene verlaufenden Fixationsbohrungen und der Ebene betragsmäßig gleich ist, die Fixationsbohrungen jedoch nach entgegengesetzten Seiten der Ebene weisen. Eine solche Anordnung hat sich als besonders günstig herausgestellt, insbesondere auch im Hinblick auf eine Durchdringung des umgebenden Gewebes mittels der Fixierelemente.

Alle Fixationsbohrungen verlaufen dabei vorzugsweise in Ebenen, die senkrecht auf der Längsachse des Fixationsnagels stehen. Es ist aber grundsätzlich auch möglich, daß diese Ebenen gegenüber der Längsachse des Fixationsnagels geneigt sind.

Die Fixierelemente können in unterschiedlicher Weise in den Fixationsbohrungen festgelegt werden, beispielsweise könnten die Fixierelemente und die Fixationsbohrungen bajonettartig miteinander verriegelt sein. In diesem Falle würden die Fixierelemente seitliche Vorsprünge tragen, die durch eine seitliche Ausnehmung in einer Fixationsbohrung bei einer bestimmten Winkelstellung des Fixierelementes hindurchpassen, die aber nach dem Hindurchtreten durch die Fixationsbohrung durch Verdrehung des Fixierelementes außen am Fixationsnagel zur Anlage kommen und dadurch eine Verriegelung bewirken.

Bei einer besonders bevorzugten Ausführungsform ist vorgesehen, daß die Fixationsbohrungen zur Festlegung der mit einem Außengewinde versehenen Fixierelemente ein Innengewinde aufweisen. In diesem Falle werden also die Fixierelemente in diese Innengewinde eingeschraubt und in der definierten Einschraublage dadurch festgelegt.

Die Innengewinde der Fixationsbohrungen sind dabei üblicherweise Gewinde, die ähnlich ausgebildet sind wie Gewinde von Knochenschrauben, so daß durch diese Fixationsbohrungen nach dem Setzen der notwendigen Bohrungen Knochenschrauben hindurchgeschraubt werden können, die sowohl in den Knochenfragmenten als auch im Fixationsnagel durch Schraubung festgelegt sind.

Dementsprechend sind auch die Gewinde der Fixierelemente den Gewinden von Knochenschrauben angepaßt, sie weisen also beispielsweise entsprechend hohe Steigungen auf. Um hier das Einschrauben zu beschleunigen, ist es besonders vorteilhaft, wenn die Innengewinde der Fixationsbohrungen Doppelgewinde sind, wenn also zwei Gewindegänge nebeneinander liegen, die um 180° in Umfangsrichtung gegeneinander versetzt sind. Dadurch wird es möglich, einerseits relativ steile Gewinde zu verwenden und dadurch ein rasches Eindrehen zu ermöglichen, andererseits aber trotzdem auf eine bestimmte Länge der Gewindebohrung eine große Zahl von Gewindegängen unterzubringen, um dadurch eine optimale Festlegung der Fixierelemente in der Fixationsbohrung zu erreichen.

Um die reproduzierbare Einschubtiefe in die Fixationsbohrungen zu gewährleisten, kann vorgesehen sein, daß die Fixierelemente die Einschubtiefe in die Innengewinde der Fixationsbohrungen begrenzende Anschläge aufweisen, diese können beispielsweise durch Verdickungen der stiftförmigen Fixierelemente gebildet werden.

Günstig ist es weiterhin, wenn die Fixierelemente im Durchtrittsbereich durch die Durchgangsbohrungen zylindrische Schäfte aufweisen, dadurch werden sie über die gesamte Länge der Durchgangsbohrungen spielfrei in diesen geführt, zur eindeutigen Festlegung des Zielgerätes ist dann nur noch eine axiale Festlegung notwendig.

Es ist weiterhin vorteilhaft, wenn der Innendurchmesser der Durchgangsbohrungen wesentlich größer ist als der Innendurchmesser der Fixationsbohrungen. Dies ermöglicht insbesondere die Verwendung der Durchgangsbohrungen als Zielbohrungen und damit das Einführen von Bohrwerkzeugen durch die Durchgangsbohrungen.

Bei einer bevorzugten Ausführungsform ist vorgesehen, daß die Fixierelemente an der fixationsnagelseitigen Außenfläche des Zielgeräts anlegbare, entfernbare Anschläge tragen. Diese Anschläge müssen deswegen entfernbar sein, weil sonst ein Fixierelement nicht aus einer Durchgangsbohrung herausgezogen werden kann, andererseits werden diese Anschläge benötigt, um eine axiale Festlegung des Zielgerätes auf dem Fixierelement zu ermöglichen.

Beispielsweise können die Anschläge durch in Vertiefungen des Fixierelementes seitlich einschiebbare Vorsprünge gebildet werden, beispielsweise sind hier seitlich einsetzbare Stifte denkbar.

Bei einer besonders bevorzugten Ausführungsform ist vorgesehen, daß die Vorsprünge die Zinken eines gabelförmigen Anschlages sind, die in eine Umfangsnut des Fixierelementes einschiebbar sind. Auf diese Weise ist es auch sehr einfach möglich, diesen Anschlag zu entfernen, es genügt nämlich, die Zinken seitlich aus der Umfangsnut herauszuziehen.

Weiterhin ist es vorteilhaft, wenn die Fixierelemente an der fixationsnagelfernen Außenfläche des Zielgerätes mittels einer auf das Fixierelement aufgeschraubten Spannmutter anliegen, die axiale Fixierung kann dann einfach durch Festdrehen dieser Spannmutter erfolgen, wobei das Zielgerät zwischen dem entfernbaren Anschlag und der Spannmutter eingespannt wird.

Bei einer bevorzugten Ausführungsform ist vorgesehen, daß das Zielgerät einen zentralen Pfosten mit Durchgangsbohrungen und seitlich von diesem abstehende Arme aufweist, die Aufnahmehülsen zur Ausbildung von Durchgangsbohrungen tragen. Auf diese Weise können auch winkelmäßig verdrehte Fixationsbohrungen mit mit diesen ausgerichteten Durchgangsbohrungen kombiniert werden.

Es ist vorteilhaft, wenn am Zielgerät weitere Durchgangsbohrungen angeordnet sind, die bei der bestimmten Position des Zielgerätes relativ zum Fixationsnagel mit Querbohrungen im Fixationsnagel ausgerichtet sind, die kein Innengewinde aufweisen. Durch Einführen von Fixierelementen in diese Querbohrungen kann in der gleichen Weise eine zusätzliche Fixierung des Zielgerätes erfolgen, allerdings lediglich gegen eine Verschwenkung, nicht dagegen hinsichtlich des Abstandes vom Fixationsnagel.

Es ist dabei günstig, wenn diese Querbohrungen im mittleren Teil des Fixationsnagels angeordnet sind.

Die Querbohrungen können in derselben Ebene liegen, die von zwei der Fixationsbohrungen aufgespannt wird.

Die nachfolgende Beschreibung bevorzugter Ausführungsformen der Erfindung dient im Zusammenhang mit der Zeichnung der näheren Erläuterung. Es zeigen:

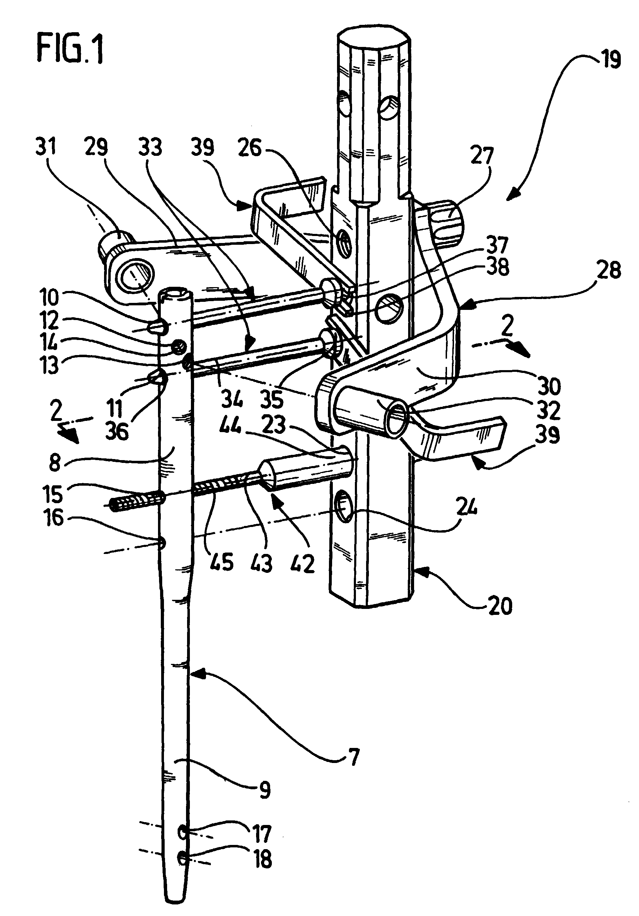

- Figur 1:: eine perspektivische Darstellung des Repositionsinstrumentariums mit einem Zielgerät, einem Fixationsnagel und mehreren Fixierelementen;
- Figur 2:: eine Schnittansicht längs Linie 2-2 in Figur 1 unter zusätzlicher Darstellung eines Bohrwerkzeuges;
- Figur 3:: eine Darstellung ähnlich Figur 2 nach dem Festlegen eines ersten Knochenfragmentes am Fixationsnagel durch Einschrauben einer Knochenschraube;
- Figur 4:: eine Schnittansicht längs Linie 4-4 in Figur 3;
- Figur 5:: eine Ansicht ähnlich Figur 3 nach Festlegung von zwei Knochenfragmenten mittels Knochenschrauben;
- Figur 6:: eine Schnittansicht längs Linie 6-6 in Figur 5 und

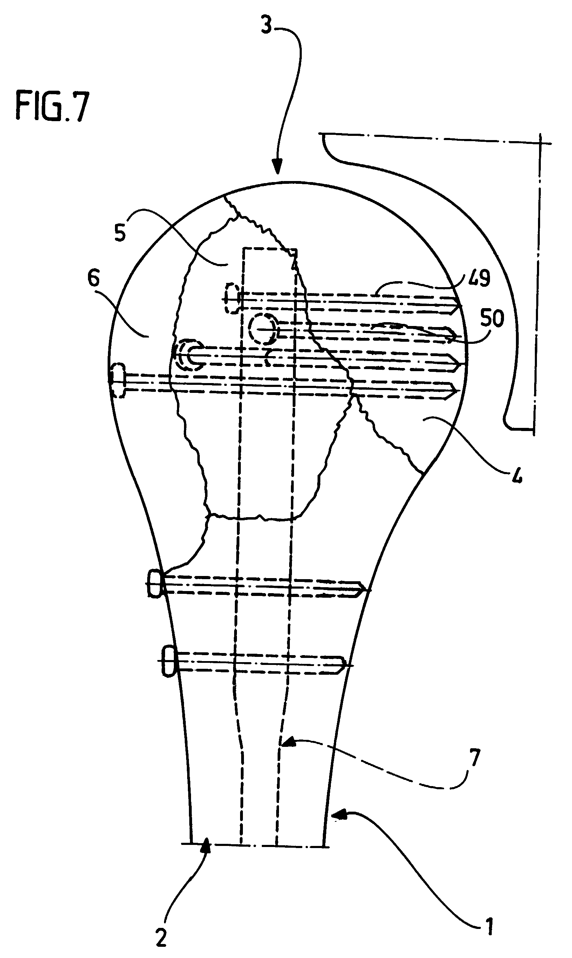

- Figur 7:: eine schematische Seitenansicht eines vollständig repositionierten Gelenkkopfes mit mehreren über Knochenschrauben am Fixationsnagel festgelegten Knochenfragmenten.

Die Erfindung wird nachstehend am Beispiel der Reposition eines Humerusknochens 1 erörtert, der einen röhrenförmigen Mittelteil 2 und einen kugelförmigen Kopf 3 aufweist. Dieser Kopf 3 soll mittels des Instrumentariums der vorliegenden Erfindung aus mehreren Knochenfragmenten 4, 5, 6 repositioniert werden. Grundsätzlich kann das beschriebene Instrumentarium auch bei anderen Knochen eingesetzt werden, die ähnlich aufgebaut sind.

Das zu diesem Zweck verwendete Instrumentarium umfaßt einen schaftförmigen, geradlinigen Fixationsnagel 7, der im wesentlichen einen proximalen kreiszylindrischen Abschnitt aufweist, der etwa in der Mitte des Fixationsnagels 7 in einen ebenfalls kreiszylindrischen distalen Abschnitt geringeren Außendurchmessers übergeht.

Im proximalen Teil des proximalen Abschnittes 8 befinden sich zwei parallel zueinander verlaufende, den proximalen Abschnitt 8 diametral durchsetzende Fixationsbohrungen 10, 11, von denen die obere Fixationsbohrung 10 relativ dicht am proximalen Ende des Fixationsnagels 7 angeordnet ist, die untere Fixationsbohrung 11 dagegen In einem Absand, der etwa einem Viertel des proximalen Abschnittes 8 entspricht.

Zwischen den beiden Fixationsbohrungen 10 und 11 sind zwei weitere den Fixationsnagel 7 diametral durchsetzende Fixationsbohrungen 12 und 13 angeordnet, die gegenüber der Ebene, die durch die Fixationsbohrungen 10 und 11 aufgespannt wird, um gleiche Winkel nach entgegengesetzten Seiten verdreht sind, dieser Winkel kann beispielsweise in der Größenordnung von 70° liegen.

Die Fixationsbohrungen 10, 11, 12, 13 tragen jeweils ein Innengewinde 14, das als Doppelgewinde ausgebildet sein kann, also unter Verwendung von zwei parallelen Schraubengängen, die in Umfangsrichtung um 180° gegeneinander versetzt sind.

Am distalen Ende des proximalen Abschnittes 8 sind in derselben Ebene, in der die Fixationsbohrungen 10 und 11 liegen, zwei weitere Querbohrungen 15, 16 im Fixationsnagel 7 angeordnet, die diesen ebenfalls diametral durchsetzen, die jedoch kein Innengewinde tragen, sondern eine glatte Innenwand aufweisen.

Schließlich sind auch am distalen Ende des distalen Abschnittes 9 zwei weitere Querbohrungen 17, 18 im Fixationsnagel 7 vorgesehen, die in derselben Ebene liegen und den Fixationsnagel 7 diametral durchsetzen, diese Ebene ist jedoch gegenüber der Ebene, die von den Fixationsbohrungen 10 und 11 aufgespannt wird, verdreht.

Das Instrumentarium umfaßt weiterhin ein Zielgerät 19 mit einem Pfosten 20, der diametral von mehreren Durchgangsbohrungen 21, 22, 23, 24 durchsetzt wird, die alle in einer Ebene liegen und deren Ausrichtung und Abstand so gewählt ist, daß diese Durchgangsbohrungen mit den Fixationsbohrungen 10 bzw. 11 und den Querbohrungen 15 bzw. 16 im Fixationsnagel 7 ausrichtbar sind.

Die genannten Durchgangsbohrungen 21, 22, 23, 24 weisen eine glatte Innenwand auf und einen Innendurchmesser, der deutlich größer ist als der Innendurchmesser der Fixationsbohrungen 10, 11, 12, 13 und der Querbohrungen 15, 16, 17, 18.

An dem Pfosten 20 ist über eine in eine Innengewindebohrung 26 eingeschraubte Spannschraube 27 ein Querträger 28 gehalten, der in zwei seitlich vom Pfosten 20 abstehenden Armen 29, 30 endet. An den freien Enden dieser Arme 29 und 30 sind Durchsteckhülsen 31, 32 angeordnet, deren Längsachsen gegenüber den Längsachsen der Durchgangsbohrungen 21, 22, 23, 24 denselben Winkel einnehmen, wie die Fixationsbohrungen 12, 13 gegenüber den Fixationsbohrungen 10, 11. Es ist dadurch möglich, den Pfosten 20 relativ zum Fixationsnagel 7 in einem Abstand anzuordnen, bei dem die Fixationsbohrungen 10, 11, 12, 13 mit den Durchsteckbohrungen 21 und 22 sowie den ebenfalls Durchsteckbohrungen bildenden Durchsteckhülsen 31, 32 jeweils ausgerichtet sind. Dabei befinden sich die Durchsteckhülsen 31 und 32 auf der jeweiligen Höhe der Fixationsbohrungen 12 bzw. 13.

Diese spezielle Position wird nachstehend als Sollposition beschrieben, es handelt sich nämlich hierbei um die Position, die das Zielgerät 19 relativ zum Fixationsnagel 7 dauerhaft einnehmen soll.

Um dies zu erreichen, werden stiftförmige Fixierelemente 33 verwandt, die einen vorderen zylindrischen Abschnitt 34 mit geringem Außendurchmesser und einen ebenfalls zylindrischen hinteren Abschnitt 35 mit grösserem Außendurchmesser aufweisen. Am vorderen Ende trägt der vordere Abschnitt 34 ein Außengewinde 36, welches als Doppelgewinde ausgebildet ist und in das Innengewinde 14 der Fixationsbohrungen 10, 11, 12, 13 eingeschraubt werden kann. Die Einschraubtiefe wird dabei durch die Länge des Außengewindes 36 begrenzt, d.h. bei vollständigem Einschrauben des Fixierelementes 33 in eine Fixationsbohrung 10 wird eine definierte Einschraubtiefe erreicht.

Die hinteren Abschnitte 35 entsprechen mit ihrem Außendurchmesser dem Innendurchmesser der Querbohrungen 15 und 16 sowie der Durchsteckhülsen 31 und 32, so daß die Fixierelemente 33 jeweils von der fixationsnagelfernen Seite durch dies Zielgerät 19 hindurchgesteckt und in die entsprechenden ausgerichteten Fixationsbohrungen 10, 11 eingeschraubt werden können und dann spielfrei in den Querbohrungen 15, 16 bzw. den Durchsteckbohrungen 31, 32 geführt sind.

Die hinteren Abschnitte 35 tragen an ihrem vorderen Ende jeweils eine Umfangsnut 37, in die die Zinken 38 eines gabelförmigen Abstandshalters 39 seitlich so einschiebbar sind, daß die Zinken 38 auf gegenüberliegenden Seiten in die Umfangsnut 37 eintauchen.

Am hinteren Ende des hinteren Abschnittes 35 ist auf ein Außengewinde 40 eine Spannmutter 41 aufschraubbar, durch die ein Fixierelement 33 in axialer Richtung relativ zu dem Pfosten 20 bzw. relativ zu den Durchsteckhülsen 31, 32 festlegbar ist. Beim Spannen der Spannmutter 41 wird nämlich der Abstandshalter 39 gegen die gegenüberliegende Seite des Pfostens bzw. der Durchsteckhülsen 31 und 32 gespannt, so daß auf diese Weise der Pfosten 20 bzw. die Durchsteckhülsen 31, 32 in genau festgelegtem Abstand von dem Fixationsnagel 7 angeordnet werden, in den die Fixierelemente 33 vollständig eingeschraubt sind.

Es ist dadurch möglich, das Zielgerät 19 relativ zum Fixationsnagel 7 exakt zu positionieren, und diese Position entspricht genau der Sollposition, in der die Fixationsbohrungen sowie die Durchgangsbohrungen und Durchsteckhülsen miteinander ausgerichtet sind.

Neben den Fixierelementen 33 werde noch Fixierstifte 42 eingesetzt, die ähnlich wie die Fixierelemente 33 einen vorderen Abschnitt 43 und einen hinteren Abschnitt 44 aufweisen, wobei der vordere Abschnitt 43 mit einem Außengewinde 45 versehen ist, das dem Außengewinde einer Knochenschraube entsprechen kann. Allerdings ist das Außengewinde 45 bei diesen Fixierstiften 42 länger ausgebildet als bei den Fixierelementen 33, außerdem fehlen bei den Fixierstiften 42 eine Umfangsnut zur Aufnahme eines Abstandshalters und ein Außengewinde zur Aufnahme einer Spannmutter.

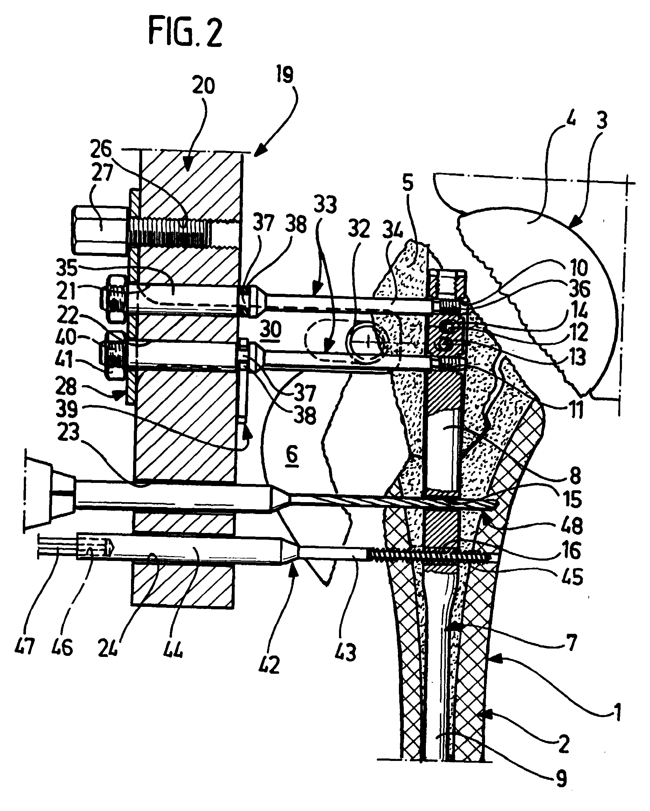

Sowohl die Fixierelemente 33 als auch die Fixierstifte 42 können zum Zwecke des Einschraubens an ihrem dem Außengewinde 36 bzw. 45 gegenüberliegenden Ende eine Aufnahmebohrung 46 für ein Eindrehwerkzeug 47 aufweisen, dies ist lediglich an einem Beispiel in Figur 2 dargestellt.

Um mit Hilfe des beschriebenen Instrumentariums die Knochenfragmente 4, 5, 6 des Humerusknochens 1 zu repositionieren, wird zunächst der Fixationsnagel 7 in das röhrenförmige Mittelteil 2 des Humerusknochens 1 eingeschoben, wobei die Fixationsbohrungen 10, 11, 12, 13 im Bereich des Kopfes 3 positioniert werden. Zwischen den Knochenfragmenten 4, 5, 6 hindurch werden dann Fixierelemente 33 in die beiden Fixationsbohrungen 10 und 11 eingeschraubt, und zwar vollständig.

Auf diese beiden Fixierelemente 33 wird das Zielgerät 19 so aufgeschoben, daß die Durchgangsbohrungen 21 und 22 die beiden Fixierelemente 33 aufnehmen, diese werden durch seitliches Einschieben von Abstandshaltern 39 in die entsprechenden Umfangsnuten 37 und durch Aufschrauben von Spannmuttern 41 in axialer Richtung am Zielgerät 19 festgelegt. Dadurch ist das Zielgerät 19 in der Sollposition positioniert. Das Zielgerät 19 kann auch am Fixationsnagel 7 befestigt werden, indem die Fixierelemente 33 durch die Durchgangsbohrungen 21, 22 oder die Hülsen 31, 32 in den Fixationsnagel eingeschraubt werden. Diese Vorgehensweise kommt dann in Frage, wenn das Zielgerät nicht auf die Fixierelemente 33 aufgeschoben werden kann, weil primär die schrägen Fixationslöcher 12, 13 verwendet werden sollen.

Zur zusätzlichen Sicherung werden zwei Fixierstifte 42 durch die Durchgangsbohrung 23, 24 hindurchgeführt und mit ihrem Außengewinde 45 durch das Mittelteil 2 des Humerusknochens 1 hindurch und durch Querbohrungen 15 und 16 hindurchgeschraubt. Zu diesem Zweck wird im Mittelteil 2 eine Knochenbohrung vorbereitet, und zwar mit Hilfe eines Bohrwerkzeuges 48, welches durch eine als Bohrlehre wirkende Durchgangsbohrung 23 hindurchgeschoben wird und die Knochenbohrung dann genau ausgerichtet mit der Querbohrung 15 im Fixationsnagel 7 erzeugt (Figur 2).

In gleicher Weise wird bei der Querbohrung 16 und der Durchgangsbohrung 24 vorgegangen.

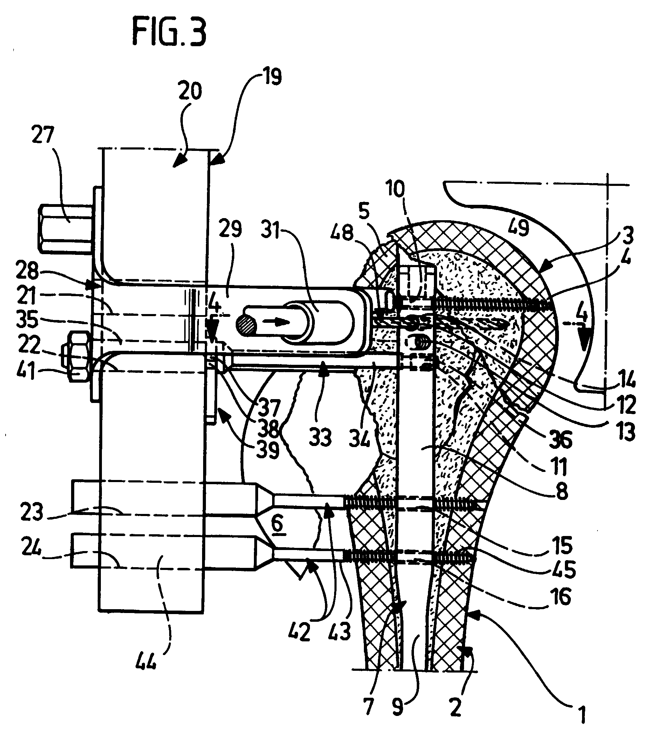

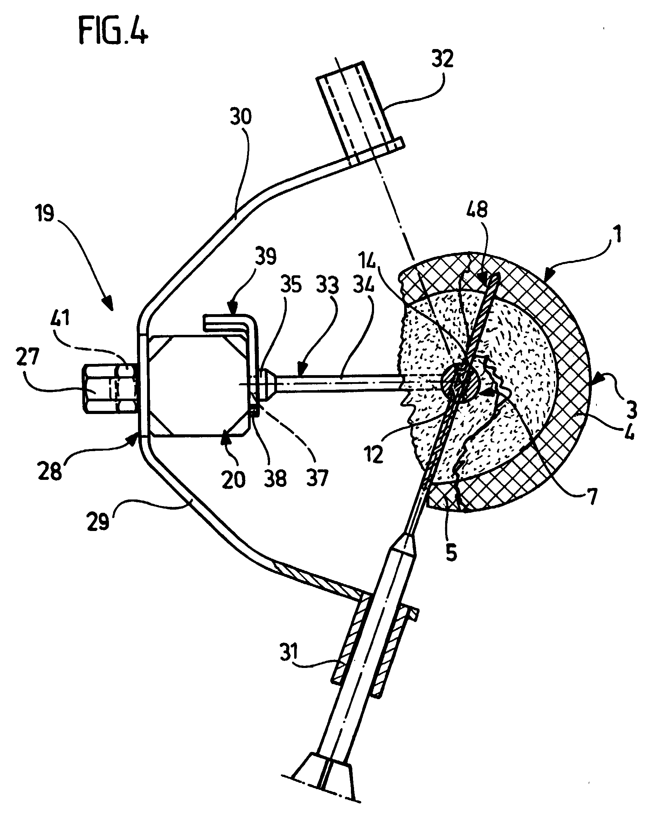

Nunmehr kann mit der Positionierung der ersten Knochenfragmente 4, 5 begonnen werden. Diese werden dazu relativ zum Fixationsnagel 7 so angeordnet, wie sie später an diesem festgelegt werden sollen. Dann wird mittels des Bohrwerkzeuges 48, welches durch die Durchsteckhülse 31 und die mit ihr ausgerichtete Fixationsbohrung 12 hindurchgesteckt wird, eine Bohrung zur Aufnahme einer Knochenschraube in die Knochenfragmente 4, 5 eingebracht, danach können die Knochenfragmente 4, 5 durch Eindrehen einer Knochenschraube 49 in die auf diese Weise hergestellte Bohrung am Fixationsnagel 7 festgelegt werden. Dieser Vorgang ist in den Figuren 3 und 4 veranschaulicht.

Eine weitere Knochenschraube soll dann beispielsweise durch die obere Fixationsbohrung 10 hindurchgeführt werden. Dazu muß zunächst das in dieser Fixationsbohrung 10 festgelegte Fixierelement 33 entfernt werden, dies erfolgt durch Lösen der entsprechenden Spannmutter 41 und durch Abnahme des entsprechenden Abstandshalters 39, das Fixierelement 33 kann dann aus der Fixationsbohrung 10 herausgeschraubt und anschließend durch die Durchgangsbohrung 21 aus dem Pfosten 20 des Zielgeräts 19 herausgezogen werden. Zur sicheren Festlegung des Zielgerätes 19 wird das in dieser Weise entnommene Fixierelement 33 anschließend beispielsweise in die Fixationsbohrung 13 eingeschraubt, zu diesem Zweck wird es zunächst durch die Durchsteckhülse 32 hindurchgeschoben und dann nach dem Einschrauben in die Fixationsbohrung 13 durch Einsetzen eines Abstandshalters 39 und durch Aufschrauben einer Spannmutter 41 in axialer Richtung festgelegt, so daß wieder das Zielgerät 19 über zwei Fixierelemente 33 sicher mit dem Fixationsnagel 7 verbunden ist. Durch die dann frei gewordene Fixationsbohrung 10 kann nunmehr nach entsprechender Positionierung eines weiteren Knochenfragmentes 6 eine neue Bohrung gesetzt werden, die durch die Fixationsbohrung 10 hindurchführt, und in diese Knochenbohrung kann eine weitere Knochenschraube 50 eingeschraubt werden.

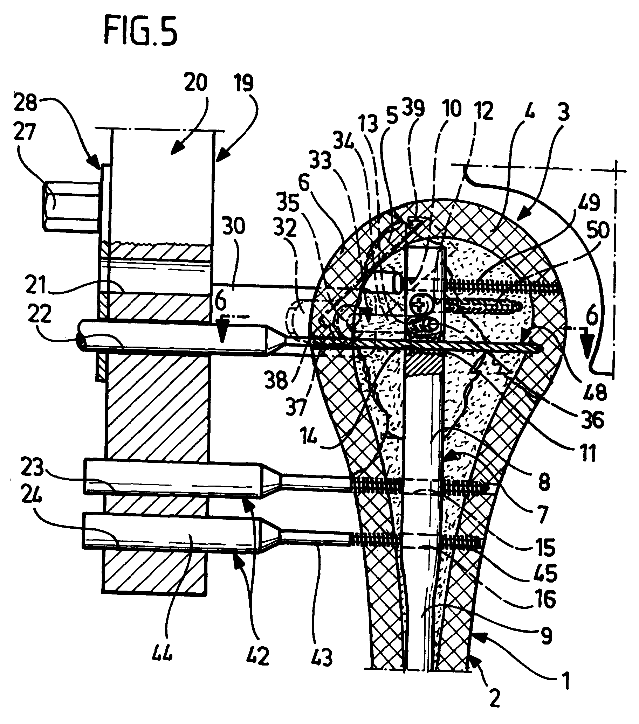

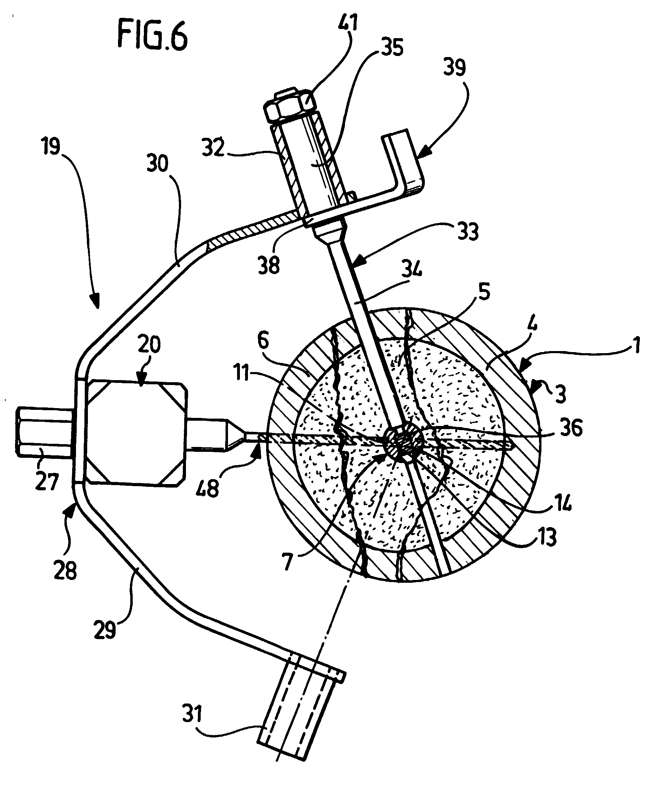

Die weiteren Bohrungen für Knochenschrauben, die durch die Fixationsbohrungen 11 und 13 hindurchgesteckt werden sollen, können dann in der gleichen Weise ausgeführt werden, wobei dabei störende Fixierelemente 33 abgenommen werden. Die Fixierung des Zielgerätes 19 erfolgt dann nur noch durch ein Fixierelement 33 und die beiden Fixierstifte 42 (Figuren 5 und 6).

Schließlich können alle auf diese Weise hergestellten Knochenbohrungen mit Knochenschrauben besetzt werden, so daß die einzelnen Knochenfragmente 4, 5, 6 optimal mit dem Fixationsnagel 7 verschraubt sind, eine zusätzliche Verschraubung des Fixationsnagels 7 mit dem Mittelteil 2 des Humerusknochens 1 kann dann ebenfalls noch erfolgen, die dazu notwendigen Gewindebohrungen sind bereits durch die Fixierstifte 42 vorbereitet.

## Patentansprüche

1. Repositionsinstrumentarium für Fragmentfrakturen von Röhrenknochen mit einem in den Röhrenknochen einführbaren, schaftförmigen, querverlaufende Fixationsbohrungen (10, 11, 12, 13) aufweisenden Fixationsnagel (7) und mit einem mit diesem über Fixierelemente (33) verbindbaren Zielgerät (19), dadurch gekennzeichnet, daß in mehrere Fixationsbohrungen (10, 11, 12, 13) stiftförmige Fixierelemente (33) reproduzierbar weit einsetzbar und in dieser Stellung festlegbar sind, daß das Zielgerät (19) zur im wesentlichen spielfreien Aufnahme derartiger Fixierelemente (33) mehrere Durchgangsbohrungen (21, 22, 31, 32) aufweist, daß die Fixationsbohrungen (10, 11, 12, 13) und die Durchgangsbohrungen (21, 22, 31, 32) so angeordnet sind, daß bei einer bestimmten Position des Zielgerätes (19) relativ zum Fixationsnagel (7) gleichzeitig jeweils eine Fixationsbohrung (10, 11, 12, 13) und eine Durchgangsbohrung (21, 22, 31 bzw. 32) miteinander ausgerichtet sind, daß die Fixierelemente (33) in den Durchgangsbohrungen (21, 22, 31, 32) in axialer Richtung derart festlegbar sind, daß das Zielgerät (19) bei in die Fixationsbohrungen (10, 11, 12, 13) eingesetzten und in diesen festgelegten Fixierelementen (33) in dieser bestimmten Position relativ zum Fixationsnagel (7) fixierbar ist, und daß die stiftförmigen Fixierelemente (33) nach Lösung dieser Fixierung und Lösung der Festlegung in den Fixationsbohrungen (10, 11, 12, 13) aus den Durchgangsbohrungen (21, 22, 31, 32) des Zielgerätes (19) herausziehbar sind.

2. Instrumentarium nach Anspruch 1, dadurch gekennzeichnet, daß im proximalen Endbereich des Fixationsnagels (7) in verschiedenem Abstand vom proximalen Ende des Fixationsnagels (7) in unterschiedliche Richtungen weisende Fixationsbohrungen (10, 11, 12, 13) vorgesehen sind, denen jeweils eine mit ihr ausrichtbare Durchgangsbohrung (21, 22, 31 bzw. 32) des Zielgerätes (19) zugeordnet ist.

3. Instrumentarium nach Anspruch 2, dadurch gekennzeichnet, daß zwei der Fixationsbohrungen (10, 11) in einer Ebene liegen und die anderen beiden Fixationsbohrungen (12, 13) schräg zu dieser Ebene verlaufen.

4. Instrumentarium nach Anspruch 3, dadurch gekennzeichnet, daß der Winkel zwischen den schräg zu der Ebene verlaufende Fixationsbohrungen (12, 13) und der Ebene betragsmäßig gleich ist, die Fixationsbohrungen (12, 13) jedoch nach entgegengesetzten Seiten der Ebene weisen.

5. Instrumentarium nach einem der voranstehenden Ansprüche, dadurch gekennzeichnet, daß alle Fixationsbohrungen (10, 11, 12, 13) in Ebenen verlaufen, die senkrecht auf der Längsachse des Fixationsnagels (7) stehen.

6. Instrumentarium nach einem der voranstehenden Ansprüche, dadurch gekennzeichnet, daß die Fixationsbohrungen (10, 11, 12, 13) zur Festlegung der mit einem Außengewinde (36) versehenen Fixierelemente (33) ein Innengewinde (14) aufweisen.

7. Instrumentarium nach einem der voranstehenden Ansprüche, dadurch gekennzeichnet, daß die Innengewinde (14) der Fixationsbohrungen (10, 11, 12, 13) Doppelgewinde sind.

8. Instrumentarium nach einem der voranstehenden Ansprüche, dadurch gekennzeichnet, daß die Fixierelemente (33) die Einschraubtiefe ihrer Gewinde (36) und das Innengewinde (14) der Fixationsbohrungen (10, 11, 12, 13) begrenzende Anschläge aufweisen.

9. Instrumentarium nach einem der voranstehenden Ansprüche, dadurch gekennzeichnet, da die Fixierelemente (33) im Durchtrittsbereich durch die Durchgangsöffnungen (21, 22, 31, 32) zylindrische Schäfte aufweisen.

10. Instrumentarium nach einem der voranstehenden Ansprüche, dadurch gekennzeichnet, daß der Innendurchmesser der Durchgangsbohrungen (21, 22, 31, 32) wesentlich größer ist als der Innendurchmesser der Fixationsbohrungen (10, 11, 12, 13).

11. Instrumentarium nach einem der voranstehenden Ansprüche, dadurch gekennzeichnet, daß die Fixierelemente (33) an der fixationsnagelseitigen Außenfläche des Zielgeräts (19) anlegbare, entfernbare Anschläge (39) tragen.

12. Instrumentarium nach Anspruch 11, dadurch gekennzeichnet, daß die Anschläge (39) durch in Vertiefungen (37) des Fixierelementes (33) seitlich einschiebbare Vorsprünge (38) sind.

13. Instrumentarium nach Anspruch 12, dadurch gekennzeichnet, daß die Vorsprünge die Zinken (38) eines gabelförmigen Anschlages (39) sind, die in eine Umfangsnut (37) des Fixierelementes (33) einschiebbar sind.

14. Instrumentarium nach einem der Ansprüche 11 bis 13, dadurch gekennzeichnet, daß die Fixierelemente (33) an der fixationsnagelfernen Außenfläche des Zielgerätes (19) mittels einer auf das Fixierelement (33) aufgeschraubten Spannmutter (41) anliegen.

15. Instrumentarium nach einem der voranstehenden Ansprüche, dadurch gekennzeichnet, daß das Zielgerät (19) einen zentralen Pfosten (20) mit Durchgangsbohrungen (21, 22) und seitlich von diesem abstehende Arme (29, 30) aufweist, die Aufnahmehülsen (31, 32) zur Ausbildung von Durchgangsbohrungen tragen.

16. Instrumentarium nach einem der voranstehenden Ansprüche, dadurch gekennzeichnet, daß am Zielgerät (19) weitere Durchgangsbohrungen (23, 24) angeordnet sind, die bei der bestimmten Position des Zielgerätes (19) relativ zum Fixationsnagel (7) mit Querbohrungen (15, 16) im Fixationsnagel (7) ausgerichtet sind, die kein Innengewinde aufweisen.

17. Instrumentarium nach Anspruch 16, dadurch gekennzeichnet, daß diese Querbohrungen (15, 16) im mittleren Teil des Fixationsnagels (7) angeordnet sind.

18. Instrumentarium nach einem der Ansprüche 3 bis 17, dadurch gekennzeichnet, daß die Querbohrungen (15, 16) in derselben Ebene liegen, die von zwei der Fixationsbohrungen (10, 11) aufgespannt wird.
